# EUROPEAN PATENT APPLICATION

(11) **EP 4 410 782 A1**
(43) Date of publication of application: **07.08.2024**
(21) Application number: 22876436.1
(22) Date of filing: 29.09.2022
(51) Int. Cl.: C07D 331/02, C08G 59/14, C08G 75/08, G02B 1/04

(54) **MONOMER FOR OPTICAL MEMBER, POLYMERIZABLE COMPOSITION FOR OPTICAL MEMBER, CURED PRODUCT, EYEGLASS LENS, AND METHOD FOR PRODUCING MONOMER FOR OPTICAL MEMBER**

(30) Priority: 30.09.2021 JP 2021160642
(71) Applicant: Hoya Lens Thailand Ltd., Pathumthani 12130 (TH)
(72) Inventor: KOUSAKA, Masahisa, Tokyo 1608347 (JP)
(74) Representative: Kraus & Lederer PartGmbB
(86) International application number: PCT/JP2022/036402
(87) International publication number: WO 2023/054572

(57) **Abstract**

One embodiment according to the present disclosure relates to a monomer for an optical component, including: a compound represented by the formula (1), in which a pH obtained through a pH measurement method below is 4.0 to 7.5.

(pH Measurement method)

80 g of a sample, 80 mL of toluene, and 250 mL of water are mixed together in a liquid separation funnel, an aqueous phase is separated off, and the pH of the aqueous phase is measured.

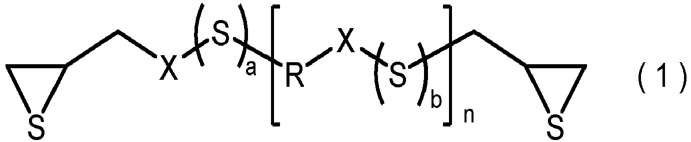

## Description

### [Technical Field]

The present disclosure relates to a monomer for an optical component, a polymerizable composition for an optical component, a cured product, a spectacle lens, and a method for producing a monomer for an optical component.

### [Background Art]

Epithio compounds are known as monomers for optical components used in spectacle lenses. By polymerizing and curing an epithio compound, a resin with high transparency, a high refractive index, and a high Abbe number can be obtained (for example, PTL 1).

### [Citation List]

### [Patent Literature]

[PTL 1] Japanese Patent Application Publication No. H10-298287

### [Summary of Invention]

### [Technical Problem]

For example, if a polymerizable composition for an optical component containing the epithio compound shown in PTL 1 is thermally polymerized, curing may be insufficient and a soft solid may be obtained, and therefore, improvement in polymerizability may be required.

One embodiment of the present disclosure relates to a monomer for an optical component with excellent polymerizability, a polymerizable composition for an optical component containing the monomer, a cured product thereof, a spectacle lens containing the cured product, and a method for producing a monomer for an optical component.

### [Solution to Problem]

The present inventors have found that polymerizability is improved by using a monomer for an optical component that exhibits a pH within a predetermined range.

One embodiment according to the present disclosure relates to a monomer for an optical component, including:
a compound represented by the formula (1): 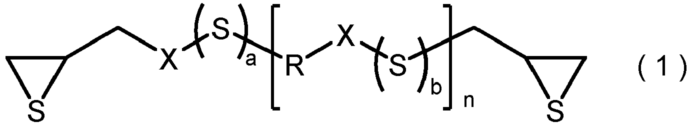
(in the formula, X is S or O, a is an integer of 0 or 1, R is a divalent hydrocarbon having 1 to 10 carbon atoms, b is an integer of 0 or 1, and n is an integer of 0 to 2),
in which a pH obtained through a pH measurement method below is 4.0 to 7.5.

### (pH Measurement method)

80 g of a sample, 80 mL of toluene, and 250 mL of water are mixed together in a liquid separation funnel, an aqueous phase is separated off, and the pH of the aqueous phase is measured.

One embodiment according to the present disclosure relates to a polymerizable composition for an optical component containing the above-described monomer.

One embodiment according to the present disclosure relates to a cured product of the above-described polymerizable composition for an optical component.

One embodiment according to the present disclosure relates to a spectacle lens including: a lens substrate containing the above-described cured product.

One embodiment according to the present disclosure relates to a method for producing a monomer for an optical component containing
a compound represented by the formula (1): 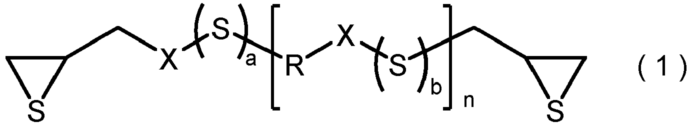
(in the formula, X is S or O, a is an integer of 0 or 1, R is a divalent hydrocarbon having 1 to 10 carbon atoms, b is an integer of 0 or 1, and n is an integer of 0 to 2), the method including:
   obtaining the compound represented by the formula (1) using a sulfurizing agent and a compound represented by the formula (2): 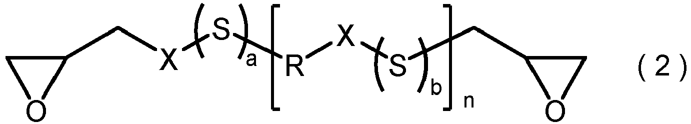 wherein, X is S or O, a is an integer of 0 or 1, R is a divalent hydrocarbon having 1 to 10 carbon atoms, b is an integer of 0 or 1, and n is an integer of 0 to 2; and
   washing the compound represented by the formula (1) with water until the pH obtained through a pH measurement method below is 4.0 to 7.5.

### (pH Measurement method)

80 g of a sample, 80 mL of toluene, and 250 mL of water are mixed together in a liquid separation funnel, an aqueous phase is separated off, and the pH of the aqueous phase is measured.

### [Advantageous Effects of Invention]

According to one embodiment of the present disclosure, it is possible to provide a monomer for an optical component with excellent polymerizability, a polymerizable composition for an optical component containing the monomer, a cured product thereof, a spectacle lens containing the cured product, and a method for producing a monomer for an optical component.

### [Description of Embodiments]

Hereinafter, embodiments of the present disclosure (hereinafter referred to as "present embodiments") will be described in detail, but the present disclosure is not limited thereto, and can be variously modified within the scope not departing from the gist thereof. In the present specification, for example, the notation of a numerical range of "1 to 100" shall encompass both the lower limit "1" and the upper limit "100". In addition, the same applies to the notation of other numerical ranges.

### [Monomer for optical component]

A monomer for an optical component according to an embodiment of the present disclosure includes
a compound represented by the formula (1): 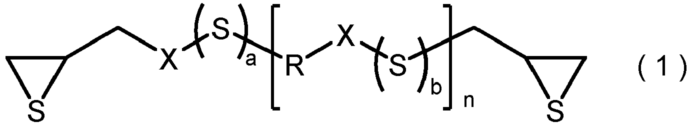
wherein, X is S or O, a is an integer of 0 or 1, R is a divalent hydrocarbon having 1 to 10 carbon atoms, b is an integer of 0 or 1, and n is an integer of 0 to 2),
in which a pH obtained through a pH measurement method below is 4.0 to 7.5.

### (pH Measurement method)

80 g of a sample, 80 mL of toluene, and 250 mL of water are mixed together in a liquid separation funnel, an aqueous phase is separated off, and the pH of the aqueous phase is measured.

According to the above-described embodiment, a monomer for an optical component with excellent polymerizability can be obtained. The excellent polymerizability of the monomer provides effects such as improving the hardness of a monomer composition after curing. In examples of the specification of the present application, the hardness after thermal polymerization is shown as one aspect of an evaluation index of polymerizability, but this is not an absolute evaluation, as other monomers, polymerization conditions, and the like may affect hardness, and differences in hardness are shown relatively, and therefore, it is determined that polymerizability is improved.

### <Compound (1)>

A monomer for an optical component according to the present embodiment includes:
a compound represented by the formula (1) (hereinafter also referred to as "compound (1)") : 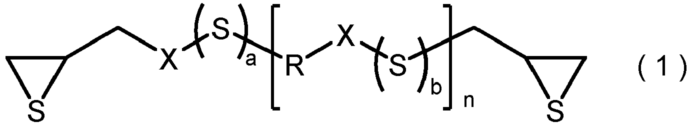
wherein, X is S or O, a is an integer of 0 or 1, R is a divalent hydrocarbon having 1 to 10 carbon atoms, b is an integer of 0 or 1, and n is an integer of 0 to 2.
X is preferably S. In a case where X is O, a is preferably 0.
The number of carbon atoms in R is preferably 1 to 4 and more preferably 2 or 3.
Examples of divalent hydrocarbons for R include an ethylene group, a propylene group, and a butylene group.

Specific examples of the compound (1) include bis(β-epithiopropyl) sulfide, bis(β-epithiopropyl) disulfide, bis(β-epithiopropyl) trisulfide, bis(β-epithiopropylthio)methane, 1,2-bis(β-epithiopropylthio)ethane, 1,3-bis(β-epithiopropylthio)propane, 1,3-bis(β-epithiopropyloxy)propane, 1,4-bis(β-epithiopropylthio)butane, and bis(β-epithiopropylthioethyl) sulfide. Among these, bis(β-epithiopropyl) sulfide and bis(β-epithiopropyl) disulfide are preferable, and bis(β-epithiopropyl) sulfide is more preferable. That is, the compound represented by the formula (1) is preferably a compound represented by the formula (1-1):

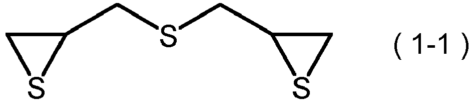

The content (also referred to as purity) of the compound (1) in the monomer for an optical component according to the present embodiment based on the total amount of the monomer for an optical component is preferably 90 mass% or more, more preferably 95 mass% or more, still more preferably 98 mass% or more, and still more preferably 99 mass% or more. The upper limit of the content of the compound (1) is not particularly limited but may be 100 mass% or less based on the total amount of the monomer for an optical component.

### (pH)

In the monomer for an optical component according to the present embodiment, a pH obtained through a pH measurement method below is 4.0 to 7.5, When the pH is within this range, a monomer for an optical component exhibiting excellent polymerizability can be obtained. The pH is preferably 4.3 to 7.5. When the pH is within the range of 4.3 to 7.5, the polymerizability is further improved, and clouding and coloration such as yellowing is further suppressed. The pH is more preferably 4.4 to 7.0, still more preferably 4.5 to 7.0, and still more preferably 4.6 to 7.0 from the viewpoints of further improving the polymerizability and further suppressing the clouding and coloration.

### (pH Measurement method)

80 g of a sample, 80 mL of toluene, and 250 mL of water are mixed together in a liquid separation funnel, an aqueous phase is separated off, and the pH of the aqueous phase is measured.

The above-described sample is a monomer for an optical component.

The pH is measured at 25°C using a pH meter (for example, manufactured by Horiba, Ltd., model number: F52, glass-bead electrode (aqueous solution type, model number: 9615S-10D)).

The pH of the monomer for an optical component according to the present embodiment can be set to be within a predetermined range, for example, by adjusting the purification conditions of the compound (1). As the purification method, the monomer can be obtained, for example, through the production method described below.

### [Method for producing monomer for optical component]

A method for producing a monomer for an optical component of the present embodiment includes: obtaining a compound represented by the formula (1) using a sulfurizing agent and a compound represented by the formula (2) (hereinafter also referred to as "compound (2)"): 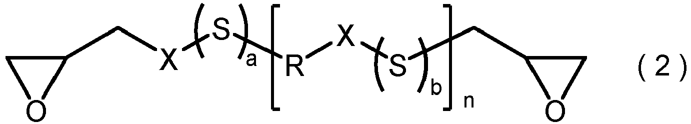
wherein, X, a, R, b, and n are the same as defined in the formula (1) (hereinafter also referred to as a "step of producing a compound (1)"); and
washing the compound represented by the formula (1) with water until the pH obtained through a pH measurement method below is 4.0 to 7.5 (hereinafter also referred to as "step of washing").

### (pH Measurement method)

80 g of a sample, 80 mL of toluene, and 250 mL of water are mixed together in a liquid separation funnel, an aqueous phase is separated off, and the pH of the aqueous phase is measured.

There may be a step of producing the compound (2) before the step of producing the compound (1).

According to the above-described embodiment, a monomer for an optical component with excellent polymerizability can be obtained.

### <Step of producing compound (2)>

The step of producing a compound (2) includes, for example, a step of reacting an epihalohydrin with a sulfur-based metal compound to obtain a compound (2).

Examples of epihalohydrins include epichlorohydrin.

Examples of sulfur-based metal compounds include metal hydrosulfides, metal sulfides, and metal polysulfides. Examples of metals include alkali metals. Among these, metal hydrosulfides are preferable, sodium hydrosulfides or potassium hydrosulfide is more preferable, and sodium hydrosulfide is still more preferable.

The molar ratio of epihalohydrin/sulfur-based metal compound is preferably 5 to 20, more preferably 5 to 15, and still more preferably 5 to 10. Here, the molar ratio of epihalohydrin/sulfur-based metal compound means a molar ratio of the amount of epihalohydrin charged to the amount of sulfur-based metal compound finally added.

A sulfur-based metal compound is preferably added to an epihalohydrin. The temperature of the epihalohydrin when adding the sulfur-based metal compound is preferably -5°C to 30°C.

A solvent may or may not be used, but is preferably used.

Examples of solvents include water, alcohols, ethers, aliphatic hydrocarbons, aromatic hydrocarbons, and halogenated hydrocarbons. Among these, water or alcohols are preferable, alcohols are more preferable, and methanol is still more preferable.

The reaction temperature is preferably -5°C to 30°C, more preferably 0°C to 20°C, and still more preferably 5°C to 15°C.

The reaction time is preferably 1 minute to 10 hours, more preferably 5 minutes to 5 hours, and still more preferably 10 minutes to 3 hours after the completion of addition of the sulfur-based metal compound.

After the above-described reaction of the epihalohydrin with the sulfur-based metal compound, a reaction is preferably caused by adding a basic compound to the reaction product obtained through the reaction.

Examples of basic compounds include amines, alkali metal salts, and alkaline earth metal salts. Among these, alkali metal salts or alkaline earth metal salts are preferable. Examples of alkali metal salts include sodium carbonate, potassium carbonate, sodium hydrogen carbonate, potassium hydrogen carbonate, sodium hydroxide, and potassium hydroxide. Examples of alkaline earth metal salts include magnesium carbonate, calcium carbonate, magnesium hydroxide, and calcium hydroxide.

The basic compound is preferably incorporated so that the molar ratio of basic compound/sulfur-based metal compound is preferably 1.0 to 3.5, more preferably 1.2 to 3.0, and still more preferably 1.5 to 3.0. Here, the molar ratio means a molar ratio of the amount of basic compound added to the amount of sulfur-based metal compound added in the previous reaction.

In the reaction with the basic compound, a solvent may or may not be used, but is preferably used.

Examples of solvents include water, alcohols, ethers, aliphatic hydrocarbons, aromatic hydrocarbons, and halogenated hydrocarbons. Among these, water or alcohols are preferable, alcohols are more preferable, and methanol is still more preferable.

In the reaction with the basic compound, the reaction temperature is preferably -5°C to 30°C, more preferably 0°C to 20°C, and still more preferably 5°C to 15°C.

The reaction time is preferably 1 minute to 10 hours, more preferably 5 minutes to 5 hours, and still more preferably 10 minutes to 3 hours after the completion of addition of the basic compound.

After the completion of the reaction, an organic solvent is preferably added thereto to extract a compound (2).

Examples of organic solvents include hydrocarbons, ethers, aliphatic hydrocarbons, aromatic hydrocarbons, and halogenated hydrocarbons. Among these, aromatic hydrocarbons or halogenated hydrocarbons are preferable, toluene, benzene, xylene, dichloromethane, and chloroform are more preferable, and toluene is still more preferable. In addition, an obtained organic phase is preferably washed with water to remove the basic compound. The organic phase is washed with water until the pH of the washing water becomes preferably 10 or less and more preferably 9 or less.

By distilling off the solvent from the organic phase thus obtained, the target compound (2) can be obtained.

### <Step of producing compound (1)>

In the step of producing a compound (1), the compound (2) and a sulfurizing agent are used to obtain a compound (1). A reaction between the compound (2) and the sulfurizing agent derivatizes an epithio group from an epoxy group of the compound (2).

Examples of sulfurizing agents include thiourea and thiocyanate. Examples of thiocyanate include sodium thiocyanate, potassium thiocyanate, ammonium thiocyanate, calcium thiocyanate, and lead thiocyanate.

The amount of sulfurizing agent used in the reaction with respect to the epoxy group of the compound (2) is preferably 1 to 5 equivalents and more preferably 1 to 3 equivalents.

The reaction temperature is, for example, 10°C to 60°C, and is preferably 10°C to 30°C when thiourea is used as a sulfurizing agent and preferably 30°C to 60°C when thiocyanate is used as a sulfurizing agent.

In the step of producing a compound (1), a solvent is preferably used. Examples of solvents include aromatic solvents, aliphatic solvents, and alcohols. Examples of aromatic solvents include toluene, xylene, chlorobenzene, dichlorobenzene, and nitrobenzene. Examples of aliphatic solvents include dichloromethane, chloroform, and dichloroethane. Examples of alcohols include methanol, ethanol, isopropanol, butanol, methoxyethanol, ethylene glycol, and glycerol.

Among these, aromatic solvents or alcohols are preferable, mixed solvents of aromatic solvents and alcohols are more preferable, and mixed solvents of toluene and methanol are still more preferable.

In the mixed solvents of aromatic solvents and alcohols, the volume ratio of aromatic solvent/alcohol is preferably 20/80 to 80/20, more preferably 30/70 to 70/30, and still more preferably 40/60 to 60/40.

In the step of producing a compound (1), a weak acid may be added for the purpose of adjusting the pH in the reaction system. Examples of weak acids include acetic acid, acetic anhydride, phosphoric acid, and phosphoric anhydride. The amount of weak acid added is preferably 0.1 to 10 volume% and more preferably 0.3 to 5 volume% based on the solvent.

### (Step of Washing)

In the step of washing, the compound (1) is washed with water until the pH obtained through the above-described pH measurement method becomes 4.0 to 7.5. By performing washing with water through the step until the pH becomes 4.0 to 7.5, a monomer for an optical component containing the compound (1) with excellent polymerizability can be obtained. Washing with water is preferably performed until the pH becomes preferably 4.3 to 7.5 from the viewpoint of obtaining a monomer for an optical component with excellent polymerizability, and becomes more preferably 4.4 to 7.0, still more preferably 4.5 to 7.0, and still more preferably 4.6 to 7.0 from the viewpoints of further improving the polymerizability and further suppressing clouding and coloration.

The cleaning step is not particularly limited, but can be performed, for example, through a method including the following steps in this order.
(i) Step of adding an aromatic solvent and an alkali metal salt aqueous solution to a reaction liquid obtained through the step of producing a compound (1) to separate an aqueous phase from an organic phase.
(ii) Step of washing the organic phase with an acidic aqueous solution.
(iii) Step of washing the organic phase with water.
(iv) Step of washing the organic phase with a sodium hydrogen carbonate aqueous solution.
(v) Step of washing the organic phase with water.

Washing in (ii) to (v) means separating the formed aqueous phase from the formed organic phase.

Impurities dissolved in the organic phase are removed by (ii), and the pH of the organic phase is made slightly acidic. Examples of acidic aqueous solutions in (ii) include a dilute sulfuric acid aqueous solution and a dilute hydrochloric acid aqueous solution. The concentration of an acidic aqueous solution is preferably 0.1 to 3 mass% and more preferably 0.3 to 2 mass%. The number of times of washing in (ii) is preferably 1 to 4 times and more preferably 2 or 3 times.

Impurities dissolved in the organic phase are removed by (iii). Examples of water in (iii) include distilled water and ion-exchanged water. The number of times of washing in (iii) is preferably 1 to 10 times, more preferably 2 to 8 times, and still more preferably 4 to 6 times.

Impurities dissolved in the organic phase are removed through (iv), and the pH of the organic phase is made slightly acidic again. The concentration of the sodium hydrogen carbonate aqueous solution is preferably 0.001 to 0.1 mass% and more preferably 0.005 to 0.05 mass%. The number of times of washing in (iv) is preferably 1 to 3 times and more preferably 1 time.

The pH of the organic phase is adjusted by (v). Examples of water in (v) include distilled water and ion-exchanged water. The number of times of washing in (v) is preferably 1 to 3 times and more preferably 1 time.

After the above-described operation, the solvent is distilled off to obtain a monomer for an optical component containing a target compound (1). Before distilling off the solvent, the organic phase and a desiccant may be mixed with each other and filtered. Examples of desiccants include sodium sulfate and magnesium sulfate.

Examples of applications of the monomer for an optical component of the present embodiment include spectacle lenses (materials for lens substrates), prisms, optical fibers, information recording substrate, and filters. Among these applications, spectacle lenses are preferable and lens substrates are more preferable.

The monomer for an optical component of the present embodiment is polymerized alone or together with other monomers to be used as an episulfide resin.

### (Episulfide resin)

The episulfide resin is a cured product of a polymerizable composition for an optical component (hereinafter also simply referred to as "polymerizable composition") containing the monomer for an optical component of the present embodiment.

The content of the monomer for an optical component of the present embodiment in the polymerizable composition is preferably 50 mass% or more, more preferably 60 mass% or more, still more preferably 70 mass% or more, still more preferably 80 mass% or more, and still more preferably 90 mass% or more. The upper limit of the content of the monomer for an optical component of the present embodiment is not particularly limited but may be 100 mass% or less, 98 mass% or less, and 96 mass% or less.

The polymerizable composition may contain other monomers. Examples of other monomers include other epithio compounds, sulfur, and polythiol compounds.

### (Other epithio compounds)

Epithio compounds are compounds having episulfide groups (epithio groups).

Examples of other epithio compounds include an episulfide compound having a linear or branched aliphatic skeleton different from the compound (1), an episulfide compound having an alicyclic skeleton, an episulfide compound having an aromatic skeleton, and an episulfide compound having a dithiane ring skeleton.

Examples of episulfide compounds having a linear or branched aliphatic skeleton different from the compound (1) include 2-(2-β-epithiopropylthioethylthio)-1,3-bis(β-epithiopropylthio)propane, 1,2-bis[(2-β-epithiopropylthioethyl)thio]-3-(β-epithiopropylthioethyl)propane, tetrakis(β-epithiopropylthiomethyl)methane, and 1,1,1-tris(β-epithiopropylthiomethyl)propane.

Examples of episulfide compounds having an alicyclic skeleton include 1,3-bis(β-epithiopropylthio)cyclohexane, 1,4-bis(β-epithiopropylthio)cyclohexane, 1,3-bis(β-epithiopropylthiomethyl)cyclohexane, 1,4-bis(β-epithiopropylthiomethyl)cyclohexane, bis[4-(β-epithiopropylthio)cyclohexyl]methane, 2,2-bis[4-(β-epithiopropylthio)cyclohexyl]propane, and bis[4-(β-epithiopropylthio)cyclohexyl]sulfide.

Examples of episulfide compounds having an aromatic skeleton include 1,3-bis(β-epithiopropylthio)benzene, 1,4-bis(β-epithiopropylthio)benzene, 1,3-bis(β-epithiopropylthiomethyl)benzene, 1,4-bis(β-epithiopropylthiomethyl)benzene, bis[4-(β-epithiopropylthio)phenyl]methane, 2,2-bis[4-(β-epithiopropylthio)phenyl]propane, bis[4-(β-epithiopropylthio)phenyl]sulfide, bis[4-(β-epithiopropylthio)phenyl]sulfine, and 4,4-bis(β-epithiopropylthio)biphenyl.

Examples of episulfide compounds having a dithiane ring skeleton include 2,5-bis(β-epithiopropylthiomethyl)-1,4-dithiane, 2,5-bis(β-epithiopropylthioethylthiomethyl)-1,4-dithiane, 2,5-bis(β-epithiopropylthioethyl)-1,4-dithiane, and 2,3,5-tri(β-epithiopropylthioethyl)-1,4-dithiane.

The content of other epithio compounds in the polymerizable composition may be 1 to 30 mass%, 5 to 20 mass%, and 8 to 15 mass%.

It is preferable that the polymerizable composition further contain sulfur or a polythiol compound in combination with an epithio compound.

The content of sulfur in the polymerizable composition is preferably 1 mass% or more, more preferably 5 to 30 mass%, and still more preferably 10 to 20 mass%.

Examples of polythiol compounds include an ester compound of a polyol compound with a mercapto group-containing carboxylic acid compound, a linear or branched aliphatic polythiol compound, a polythiol compound having an alicyclic structure, and a polythiol compound having an aromatic ring structure.

In the ester compound of a polyol compound with a mercapto group-containing carboxylic acid compound, examples of polyol compounds include a compound having two or more hydroxyl groups in its molecule.

Examples of polyol compounds include ethylene glycol, diethylene glycol, propanediol, propanetriol, butanediol, trimethylolpropane, bis(2-hydroxyethyl) disulfide, pentaerythritol, and dipentaerythritol.

Examples of mercapto group-containing carboxylic acid compounds include thioglycolic acid, mercaptopropionic acid, a thiolactic acid compound, and thiosalicylic acid.

Examples of the ester compound of a polyol compound with a mercapto group-containing carboxylic acid compound include ethylene glycol bis(2-mercaptoacetate), ethylene glycol bis(3-mercaptopropionate), diethylene glycol bis(2-mercaptoacetate), diethylene glycol bis(3-mercaptopropionate), 1,4-butanediol bis(2-mercaptoacetate), 1,4-butanediol bis(3-mercaptopropionate), trimethylolpropane tris(2-mercaptoacetate), trimethylolpropane tris(3-mercaptopropionate), pentaerythritol tetrakis(2-mercaptoacetate), pentaerythritol tetrakis(3-mercaptopropionate), dipentaerythritol hexakis(2-mercaptoacetate), and dipentaerythritol hexakis(3-mercaptopropionate).

Examples of linear or branched aliphatic polythiol compounds include 1,2-ethanedithiol, 1,1-propanedithiol, 1,2-propanedithiol, 1,3-propanedithiol, 2,2-propanedithiol, 1,6-hexanedithiol, 1,2,3-propanetrithiol, 2,2-dimethylpropane-1,3-dithiol, 3,4-dimethyloxybutane-1,2-dithiol, 2,3-dimercapto-1-propanol, 1,2-dimercaptopropyl methyl ether, 2,3-dimercaptopropyl methyl ether, 2-(2-mercaptoethylthio)propane-1,3-dithiol, 2,2-bis(mercaptomethyl)-1,3-propanedithiol, bis(mercaptomethylthio)methane, tris(mercaptomethylthio)methane, bis(2-mercaptoethylthio)methane, 1,2-bis(mercaptomethylthio)ethane, 1,2-bis(2-mercaptoethylthio)ethane, 1,3-bis(mercaptomethylthio)propane, 1,3-bis(2-mercaptoethylthio)propane, 1,1,2,2-tetrakis(mercaptoethylthio)ethane, 1,1,3,3-tetrakis(mercaptoethylthio)propane, 3-mercaptomethyl-1,5-dimercapto-2,4-dithiapentane, tetrakis(mercaptoethylthio)propane, bis(2-mercaptoethyl)ether, bis(2-mercaptoethyl) sulfide, bis(2-mercaptoethyl) disulfide, 1,2-bis(2-mercaptoethylthio)-3-mercaptopropane, 4,7-bis(mercaptomethyl)-3,6,9-trithiaundecane-1,11-dithiol, 4,8-bis(mercaptomethyl)-3,6,9-trithiaundecane-1,11-dithiol, and 5,7-bis(mercaptomethyl)-3,6,9-trithiaundecane-1,11-dithiol.

Examples of polythiol compounds having an aliphatic ring structure include 1,1-cyclohexanedithiol, 1,2-cyclohexanedithiol, methylcyclohexanedithiol, bis(mercaptomethyl)cyclohexane, 2-(2,2-bis(mercaptomethylthio)ethyl)-1,3-dithiethane, 2,5-bis(mercaptomethyl)-1,4-dithiane, and 4,8-bis(mercaptomethyl)-1,3-dithiane.

Examples of polythiol compounds having an aromatic ring structure include 1,3-dimercaptobenzene, 1,4-dimercaptobenzene, 1,3-bis(mercaptomethyl)benzene, 1,4-bis(mercaptomethyl)benzene, 1,3-bis(mercaptoethyl)benzene, 1,4-bis(mercaptoethyl)benzene, 1,3,5-trimercaptobenzene, 1,3,5-tris(mercaptomethyl)benzene, 1,3,5-tris(mercaptoethyl)benzene, 4,4'-dimercaptobiphenyl, 4,4'-dimercaptobibenzyl, 2,5-toluenedithiol, 1,5-naphthalenedithiol, 2,6-naphthalenedithiol, 2,7-naphthalenedithiol, 2,4-dimethylbenzene-1,3-dithiol, 4,5-dimethylbenzene-1,3-dithiol, 9,10-anthracenedimethanethiol, 1,3-di(p-methyloxyphenyl)propane-2,2-dithiol, 1,3-diphenylpropane-2,2-dithiol, phenylmethane-1,1-dithiol, and 2,4-di(p-mercaptophenyl)pentane.

These may be used alone or two or more thereof may be used.

When a polythiol compound is used in combination with an epithio compound, the content of the polythiol compound in the polymerizable component is preferably 2 to 50 mass%, more preferably 4 to 40 mass%, still more preferably 4 to 30 mass%, still more preferably 4 to 20 mass%, and still more preferably 4 to 10 mass%.

The polymerizable composition preferably contains a polymerization catalyst. Examples of polymerization catalysts include a nitrogen-containing compound.

Examples of nitrogen-containing compounds include tertiary amines, quaternary ammonium salts, imidazole compounds, and pyrazole compounds. Tertiary amines are preferably hindered amines.

Examples of tertiary amines include triethylamine, tri-n-propylamine, triisopropylamine, tri-n-butylamine, triisobutylamine, N,N-dimethylbenzylamine, N-methylmorpholine, N,N-dimethylcyclohexylamine, pentamethyldiethylenetriamine, bis(2-dimethylaminoethyl) ether, N-methylmorpholine, N,N'-dimethylpiperazine, N,N,N',N'-tetramethylethylenediamine, and 1,4-diazabicyclo[2.2.2]octane(DABCO).

Examples of hindered amines include 1,2,2,6,6-pentamethyl-4-piperidinol, 1,2,2,6,6-pentamethyl-4-hydroxyethyl-4-piperidinol, methyl-1,2,2,6,6-pentamethyl-4-piperidyl sebacate, a mixture of methyl-1,2,2,6,6-pentamethyl-4-piperidyl sebacate and bis(1,2,2,6,6-pentamethyl-4-piperidyl)sebacate, bis(1,2,2,6,6-pentamethyl-4-piperidyl)sebacate, bis(2,2,6,6-tetramethyl-1-(octyloxy)-4-piperidyl)sebacate, bis(1,2,2,6,6-pentamethyl-4-piperidyl)[[3,5-bis(1,1-dimethylethyl)-4-hydroxyphenyl]methyl]butylmalonate, and tetrakis(1,2,2,6,6-pentamethyl-4-piperidyl)butane-1,2,3,4-tetracarboxylate.

Examples of quaternary ammonium salts include tetraethylammonium hydroxide.

Examples of imidazole compounds include imidazole, 1-methyl-2-mercapto-1H-imidazole, 1,2-dimethylimidazole, benzylmethylimidazole, and 2-ethyl-4-imidazole.

Examples of pyrazole compounds include pyrazole and 3,5-dimethylpyrazole.

Among these, tertiary amines such as hindered amines, imidazole compounds, and pyrazole compounds are preferable, imidazole compounds are more preferable, and 1-methyl-2-mercapto-1H-imidazole is still more preferable.

The amount of polymerization catalyst added in the polymerizable composition based on the total amount of 100 parts by mass of the polymerizable component is preferably 0.001 to 2 parts by mass, more preferably 0.005 to 1 part by mass, and still more preferably 0.007 to 0.5 parts by mass.

### [Method for producing lens substrate for spectacle lens]

A lens substrate for a spectacle lens is not particularly limited but can be obtained, for example, through a production method including
a step of curing the polymerizable composition described above and
a step of annealing the cured resin.

The polymerization is preferably performed by a cast polymerization method. A lens substrate can be obtained through performing polymerization by injecting a polymerizable composition into a mold die which is a combination of a glass or metal mold and tape or a gasket.

Polymerization conditions can be appropriately set according to the polymerizable composition. The polymerization initiation temperature is preferably 0°C to 50°C and more preferably 10°C to 40°C. Preferably, the temperature is raised from the polymerization initiation temperature, and curing by heating is then performed. For example, the maximum heating temperature is usually 110°C to 130°C.

After polymerization is completed, the lens substrate may be released from the mold and subjected to an annealing treatment. The temperature of the annealing treatment is preferably 100°C to 150°C.

As described above, according to the present embodiment, a monomer for an optical component with excellent polymerizability can be obtained. In addition, according to the present embodiment, a monomer for an optical component that suppresses clouding and yellowing of a cured product can be obtained. For this reason, favorable polymerizability is obtained and clouding and yellowing are suppressed, and therefore it is suitably used as an optical material such as a lens substrate for a spectacle lens.

The present specification discloses the following embodiment.
<1> A monomer for an optical component, including: a compound represented by the formula (1): 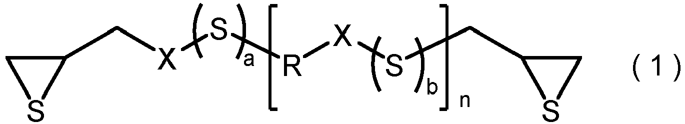
   wherein, X is S or O, a is an integer of 0 or 1, R is a divalent hydrocarbon having 1 to 10 carbon atoms, b is an integer of 0 or 1, and n is an integer of 0 to 2,
   in which a pH obtained through a pH measurement method below is 4.0 to 7.5.
   (pH Measurement method)
   80 g of a sample, 80 mL of toluene, and 250 mL of water are mixed together in a liquid separation funnel, an aqueous phase is separated off, and the pH of the aqueous phase is measured.
<2> The monomer according to <1>, in which the compound represented by the formula (1) is a compound represented by the formula (1-1): 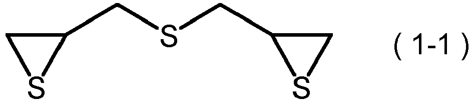
<3> The monomer according to <1> or <2>, in which the pH is 4.3 to 7.5.
<4> The monomer according to <1> or <2>, in which the pH is 4.5 to 7.0.
<5> A polymerizable composition for an optical component, including: the monomer according to any one of <1> to <4>.
<6> A cured product of the polymerizable composition for an optical component according to <5>.
<7> A spectacle lens including: a lens substrate containing the cured product according to <6>.
<8> A method for producing a monomer for an optical component containing a compound represented by the formula (1): 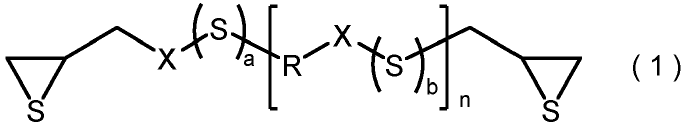 wherein, X is S or O, a is an integer of 0 or 1, R is a divalent hydrocarbon having 1 to 10 carbon atoms, b is an integer of 0 or 1, and n is an integer of 0 to 2), the method including:
   obtaining the compound represented by the formula (1) using a sulfurizing agent and a compound represented by the formula (2): 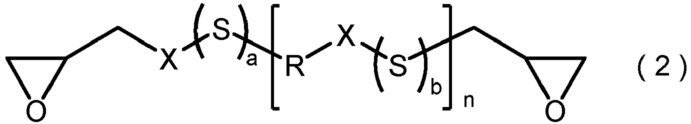
   wherein, X is S or O, a is an integer of 0 or 1, R is a divalent hydrocarbon having 1 to 10 carbon atoms, b is an integer of 0 or 1, and n is an integer of 0 to 2; and washing the compound represented by the formula (1) with water until the pH obtained through a pH measurement method below is 4.0 to 7.5.

### (pH Measurement method)

80 g of a sample, 80 mL of toluene, and 250 mL of water are mixed together in a liquid separation funnel, an aqueous phase is separated off, and the pH of the aqueous phase is measured.

### [Examples]

Hereinafter, the present embodiment will be described in more detail with reference to examples and comparative examples. The present invention is not limited to the following examples.

### [Measurement method]

### <pH>

80 g of an obtained monomer for an optical component, 80 mL of toluene, and 250 mL of water were mixed together in a liquid separation funnel, and an aqueous phase was separated off. The pH of the obtained aqueous phase was measured at 25°C using a pH meter (manufactured by Horiba, Ltd., model number: F52, glass-bead electrode, aqueous solution type, model number: 9615S-10D)).

### [Evaluation method]

### <Polymerizable compound>

Polymerizable compositions which were thermally polymerized according to the method described in the examples were observed, and their polymerizability was evaluated.

### (Evaluation criteria)

A: A hard resin was obtained by curing through thermal polymerization.
B: A resin was cured through thermal polymerization but was soft.
C: A resin was not cured even through thermal polymerization.

### <Suppression of clouding>

Polymerizable compositions were thermally polymerized according to the method described in the examples, and the degree of their cloudiness was evaluated according to the following criteria.

### (Evaluation criteria)

A: Cloudiness was not observed in a resin cured through thermal polymerization.
B: Cloudiness was observed in a resin cured through thermal polymerization, but the resin had transparency with cloudy spots scattered therethrough.
C: Cloudiness was observed in a resin cured through thermal polymerization, and the entire resin was cloudy and white.
-: A resin was not cured even through thermal polymerization.

### <Suppression of coloration>

Polymerizable compositions were thermally polymerized according to the method described in the examples, and the degree of their coloration was evaluated according to the following criteria.

### (Evaluation criteria)

A: A resin cured through thermal polymerization was colorless and transparent.
B: A resin cured through thermal polymerization was pale yellow.
C: A resin cured through thermal polymerization was yellow.

### <Example 1>

### (Production of monomer)

A solution obtained by dissolving 8.0 g of 70 mass% sodium hydrosulfide in 20 mL of methanol was slowly added dropwise to 46.4 g of epichlorohydrin while stirring at 0°C to 5°C, followed by stirring for 1 hour. Next, a solution obtained by dissolving 14.0 g of sodium hydroxide in 40 mL of water was added dropwise thereto while stirring at 5°C to 10°C, followed by stirring for 1 hour. After the completion of the reaction, extraction was performed with 200 mL of toluene, washing with 200 mL of water was repeated, and then a toluene solvent was removed to obtain bis(glycidyl) sulfide.

Next, 36.6 g of the obtained bis(glycidyl) sulfide and 39.96 g of thiourea was dissolved in 43.3 mL of toluene and 43.3 mL of methanol, and 0.52 mL of acetate was added thereto to cause a reaction at room temperature (25°C) for 16 hours. 80 mL of toluene was added to the reaction mixture, 250 mL of water in which 0.5 g of NaCl was dissolved was added thereto, an aqueous phase was separated from an organic phase using a liquid separation funnel, and the organic phase was washed twice with 150 mL of a 1 mass% sulfuric acid aqueous solution. Thereafter, the organic phase was then washed 5 times with 250 mL of water. Thereafter, the organic phase was washed once with 250 mL of 0.01 mass% sodium hydrogen carbonate aqueous solution and further washed with 250 mL of water. The organic phase was dried with anhydrous sodium sulfate and the solvent was distilled off to obtain white solid bis(β-epithiopropylthio) sulfide as a monomer for an optical component. The pH of the obtained bis(β-epithiopropylthio) sulfide was measured and shown in Table 1.

### (Polymerization)

79.92 parts by mass of bis(β-epithiopropyl) sulfide and 14.00 parts by mass of sulfur were added to a 300 mL eggplant flask and degassed for 60 minutes while heating to 60°C. Then, 0.467 parts by mass of 1-methyl-2-mercapto-1H-imidazole was added thereto, a preliminary reaction was performed at 60°C for 60 minutes while stirring in a sealed state at normal pressure, and the sample was then cooled to 20°C, and 0.13 parts by mass of dibutyltin dichloride was added to stop the preliminary reaction.

6.08 parts by mass of bis(2-mercaptoethyl) sulfide, 0.001 parts by mass of an acidic phosphoric ester, "JP506H" (trade name, manufactured by Johoku Chemical Co., Ltd.), and 0.020 parts by mass of tetrabutylphosphonium bromide were added to a separate container and mixed together and dissolved therein, this mixture was added to the pre-reacted mixture, and the resultant mixture was degassed while stirring at 20°C to form a homogeneous solution.

Next, the homogeneous solution was injected into a sample bottle (capacity of 6 cc, inner diameter of 14 mm) made of glass while filtrating the solution through a 3-micron polyethylene terephthalate filter, the temperature was raised from 30°C to 100°C over 24 hours in an oven for polymerizing and curing to obtain a resin. The obtained resin was subjected to various evaluations described above visually, and the results are shown in Table 1.

### <Example 2>

In Example 2, bis(β-epithiopropylthio) sulfide was obtained in the same manner as in Example 1 except that 43.3 mL of toluene was added to the obtained reaction mixture, 250 mL of water in which 0.5 g of NaCl was dissolved was added thereto, an aqueous phase was separated from an organic phase using a liquid separation funnel, and the organic phase was washed twice with 150 mL of a 1 mass% sulfuric acid aqueous solution and further washed 5 times with 250 mL of water, without subsequent washing with a sodium hydrogen carbonate aqueous solution and washing with water. The pH of the obtained bis(β-epithiopropylthio) sulfide was measured and shown in Table 1.

The obtained bis(β-epithiopropylthio) sulfide was polymerized through the same method as in Example 1, and various evaluations were performed and shown in Table 1.

### <Example 3>

In Example 3, bis(β-epithiopropylthio) sulfide was obtained in the same manner as in Example 1 except that 43.3 mL of toluene was added to the obtained reaction mixture, 250 mL of water in which 0.5 g of NaCl was dissolved was added thereto, an aqueous phase was separated from an organic phase using a liquid separation funnel, and the organic phase was washed twice with 150 mL of a 1 mass% sulfuric acid aqueous solution and further washed 4 times with 250 mL of water, without subsequent washing with a sodium hydrogen carbonate aqueous solution and washing with water. The pH of the obtained bis(β-epithiopropylthio) sulfide was measured and shown in Table 1.

The obtained bis(β-epithiopropylthio) sulfide was polymerized through the same method as in Example 1, and various evaluations were performed and shown in Table 1.

### <Example 4>

In Example 4, bis(β-epithiopropylthio) sulfide was obtained in the same manner as in Example 1 except that 43.3 mL of toluene was added to the obtained reaction mixture, 250 mL of water in which 0.5 g of NaCl was dissolved was added thereto, an aqueous phase was separated from an organic phase using a liquid separation funnel, and the organic phase was washed twice with 150 mL of a 1 mass% sulfuric acid aqueous solution and further washed twice with 250 mL of water, without subsequent washing with a sodium hydrogen carbonate aqueous solution and washing with water. The pH of the obtained bis(β-epithiopropylthio) sulfide was measured and shown in Table 1.

The obtained bis(β-epithiopropylthio) sulfide was polymerized through the same method as in Example 1, and various evaluations were performed and shown in Table 1.

### <Comparative Example 1>

In Comparative Example 1, bis(β-epithiopropylthio) sulfide was obtained in the same manner as in Example 1 except that 43.3 mL of toluene was added to the obtained reaction mixture, 250 mL of water in which 0.5 g of NaCl was dissolved was added thereto, an aqueous phase was separated from an organic phase using a liquid separation funnel, and the organic phase was washed twice with 150 mL of a 1 mass% sulfuric acid aqueous solution and further washed once with 250 mL of water, without subsequent washing with a sodium hydrogen carbonate aqueous solution and washing with water. The pH of the obtained bis(β-epithiopropylthio) sulfide was measured and shown in Table 1.

The obtained bis(β-epithiopropylthio) sulfide was polymerized through the same method as in Example 1, and various evaluations were performed and shown in Table 1.

### [Table 1]

**Table 1**

| | Monomer for optical component | | Evaluation | | |
|---|---|---|---|---|---|
| | Compound | pH | Polymerizability | Suppression of clouding | Suppression of coloration |
| Example 1 | Epithiopropyl sulfide | 7.0 | A | A | A |
| Example 2 | Epithiopropyl sulfide | 4.6 | A | A | A |
| Example 3 | Epithiopropyl sulfide | 4.4 | A | B | B |
| Example 4 | Epithiopropyl sulfide | 4.2 | B | C | C |
| Comparative Example 1 | Epithiopropyl sulfide | 3.4 | C | - | C |

From the above-described results, it can be seen that monomers for an optical component having a pH of 4.0 or more exhibit excellent polymerizability. In addition, it can be seen that monomers for an optical component having a pH of 4.3 or more exhibit superior polymerizability, suppress occurrence of clouding, and further suppress coloration. Furthermore, it can be seen that monomers for an optical component having a pH of 4.5 or more exhibit superior polymerizability, exhibit superior transparency, and further suppress coloration.

## Claims

1. A monomer for an optical component, comprising:
a compound represented by the formula (1): 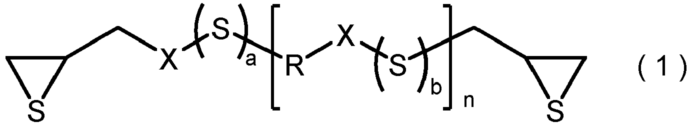
wherein, X is S or O, a is an integer of 0 or 1, R is a divalent hydrocarbon having 1 to 10 carbon atoms, b is an integer of 0 or 1, and n is an integer of 0 to 2,
wherein a pH obtained through a pH measurement method below is 4.0 to 7.5,
(pH Measurement method)
80 g of a sample, 80 mL of toluene, and 250 mL of water are mixed together in a liquid separation funnel, an aqueous phase is separated off, and the pH of the aqueous phase is measured.

2. The monomer according to claim 1,
wherein the compound represented by the formula (1) is a compound represented by the formula (1-1):

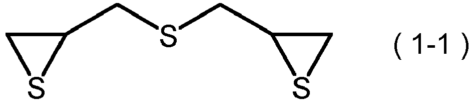

3. The monomer according to claim 1,
wherein the pH is 4.3 to 7.5.

4. The monomer according to claim 1,
wherein the pH is 4.5 to 7.0.

5. A polymerizable composition for an optical component, comprising:
the monomer according to any one of claims 1 to 4.

6. A cured product of the polymerizable composition for an optical component according to claim 5.

7. A spectacle lens comprising:
a lens substrate containing the cured product according to claim 6.

8. A method for producing a monomer for an optical component containing a compound represented by the formula (1): 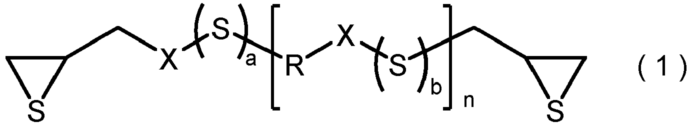 wherein, X is S or O, a is an integer of 0 or 1, R is a divalent hydrocarbon having 1 to 10 carbon atoms, b is an integer of 0 or 1, and n is an integer of 0 to 2, the method comprising:
obtaining the compound represented by the formula (1) using a sulfurizing agent and a compound represented by the formula (2): 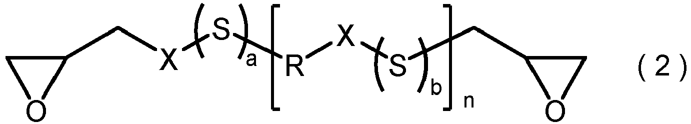
wherein, X is S or O, a is an integer of 0 or 1, R is a divalent hydrocarbon having 1 to 10 carbon atoms, b is an integer of 0 or 1, and n is an integer of 0 to 2; and
washing the compound represented by the formula (1) with water until the pH obtained through a pH measurement method below is 4.0 to 7.5,
(pH Measurement method)
80 g of a sample, 80 mL of toluene, and 250 mL of water are mixed together in a liquid separation funnel, an aqueous phase is separated off, and the pH of the aqueous phase is measured.
